(19) Europäisches Patentamt
European Patent Office
Office européen des brevets

(11) Publication number: 0 103 840
B1

(12) **EUROPEAN PATENT SPECIFICATION**

(45) Date of publication of patent specification: 04.03.87

(21) Application number: 83109037.8

(22) Date of filing: 13.09.83

(51) Int. Cl.[4]: C 07 D 249/04, C 07 D 285/06, C 07 D 249/06 // A61K31/41

# (54) Method for preparing 1,2,3-trihetero 5-membered heterocyclic compounds.

(30) Priority: 17.09.82 JP 160916/82
16.02.83 JP 22812/83

(43) Date of publication of application:
28.03.84 Bulletin 84/13

(45) Publication of the grant of the patent:
04.03.87 Bulletin 87/10

(84) Designated Contracting States:
CH DE FR GB IT LI

(56) References cited:

JOURNAL OF THE AMERICAN CHEMICAL SOCIETY, vol. 77, no. 20, 20 October 1955, Columbus Ohio, US C.D. HURD et al: "On acylhydrazones and 1,2,3-thiadiazoles", pages 5359-5364

CHEMICAL ABSTRACTS, vol. 96, no. 7, 5 February 1982, pages 615,616, Columbus, Ohio, US Abstract number 52242g, F. BELLESIA et al: "The reaction of alpha-bêta-unsaturated p-tosyl-hydrazones with sulfur chlorides"

(73) Proprietor: LEDERLE (JAPAN) LTD.
*Hattori Bldg., 5th Floor 10-3 Kyobashi 1-chome*
Chuo-ku Tokyo (JP)

(72) Inventor: Sakai, Kunikazu
No. 5-16-17, Chuorikan
Yamato-shi Kanagawa (JP)
Inventor: Tsunemoto, Daiei
No. 2020-59, Midorigaoka
Zama-shi Kanagawa (JP)
Inventor: Kobori, Takeo
No. 3-27-14, Mouridai
Atsugi-shi Kanagawa (JP)
Inventor: Kondo, Kiyoshi
No. 5-16-4, Chuorikan
Yamato-shi Kanagawa (JP)
Inventor: Hida, Nobuko
4-1-2, Kinuta
Setagaya-ku Tokyo (JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 103 840 B1

**Description**

FIELD OF THE INVENTION

The present invention relates to a novel method for preparing 1,2,3-trihetero 5-membered heterocyclic compound represented by the general formula (III):

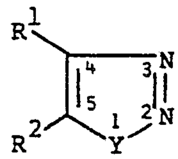

(III)

[wherein Y represents

—N— or —S—
|
R

(wherein R represents an alkyl group, a 2-alkenyl group, an aralkyl group or an aromatic group); $R^1$ represents a hydrogen atom, an alkyl group or an aromatic group, provided that when Y represents —S—, $R^1$ represents a hydogen atom; $R^2$ represents a group represented by M—Y— (where, when Y represents

—N—,
|
R

M represents a hydrogen atom, and when Y represents —S—, M represents an alkali metal atom), a hydrogen atom, an alkyl group or an atomatic group].

Particularly, the present invention comprises a novel method for preparing 5-mercapto-1,2,3-thiadiazole salts represented by the general formula (VI):

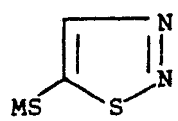

(VI)

(wherein M is an alkali metal atom); and a novel method for preparing 1,2,3-triazole derivatives represented by the general formula (IX):

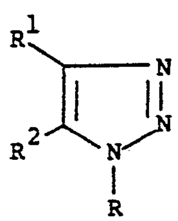

(IX)

(wherein R is an alkyl group, a 2-alkenyl group, an aralkyl group, or an aromatic group, $R^1$ is a hydrogen atom, an alkyl group or an aromatic group and $R^2$ is a hydrogen atom, an alkyl group, an aromatic group or a group represented by R—NH—).

BACKGROUND OF THE INVENTION

The 5-mercapto-1,2,3-thiadiazole salts represented by the above general formula (VI) obtainable by using the method of this invention are important compounds as modifier of cephalosporins [see G. S. Lewis and P. H. Nelson, *J. Med. Chem.*, 1979, *22*, 1214 and Japanese Patent Application (OPI) No. 59895/82 (the term "OPI" as used herein refers to a "published unexamined Japanese patent application") which corresponds to Published Unexamined British Patent Application No. 2083461 and Belgian Patent 88913].

Heretofore, the followings have been considered to be the best method for preparing 5-mercapto-1,2,3-thiadiazole salts: haloacetaldehyde is allowed to react with a semicarbazide or an alkoxycarbonylhydrazine to afford the corresponding hydrazone, then the hydrazone is converted to 5-chloro-1,2,3-thiadiazole by the action of thionyl chloride and the thiadiazole is treated with sodium sulfide to give a desired thiadiazole salt (see West German Patent Application (OLS) Nos. 2,636,994 and 2,856,404).

However, this method contains a drawback in that 5-chloro-1,2,3-thiadiazole formed as an intermediate is explosive (see S. Morisaki, *Thermochim. Acta.*, 1981, *47,* 85). Accordingly, considerable difficulties are involved with treatment and handling.

Further, the 1,2,3-triazole derivatives represented by the above general formula (IX) obtainable by using the method of this invention are useful for the intermediates of drugs and insecticides (see Japanese Patent Application (OPI) No. 127363/81). For example, benzyl group can easily be removed from N-benzyl derivatives and the resulting compounds can be converted to 5-mercapto-1,2,3-triazole which are modifiers for cephalosporin derivatives (see Japanese Patent Application (OPI) No. 108974/77 which corresponds to Belgian Patent 851,822 and West German Patent Application (OLS) No. 2,709,122). Furthermore, oximecarbamate derivatives of 1,2,3-triazole are known to be potent as insecticides, acaricides and nematocides (see Japanese Patent Application (OPI) No. 116464/77 which corresponds to Belgian Patent 852,897 and French Patent 2,345,435).

Heretofore, the followings have been known as methods for preparing 1,2,3-triazole derivatives: (a) a method of preparing them through oxidation process of a 1,2,3-triazoline derivative which is obtained by the reaction of a vinyl compound with an azide compound (see G. Beck and D. Gunther, *Chem. Ber.*, 1973, *106,* 2758); (b) a method of preparing them by basic treatment of a 1,2,3-triazoline derivative which is obtained by reacting an azide compound with a vinyl compound having an easily removable leaving group when treated with base (see Japanese Patent Application (OPI) No. 127363/81).

However, these conventional methods require azide derivatives which cannot be used in large scale production owing to their explosion possibility. Furthermore, method (a) contains some problems in that the oxidation process induces the decrease of product yields, etc.

## SUMMARY OF THE INVENTION

As a result of intensive studies on a safe and conventional method for the production of 1,2,3-trihetero 5-membered heterocyclic compound, the present inventors have achieved this invention.

The present invention comprises the production of 1,2,3-trihetero 5-membered heterocyclic compound represented by the general formula (III):

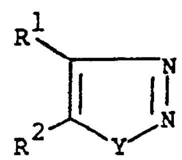

(III)

comprising the steps of:

providing a hydrazone derivative represented by the general formula (II):

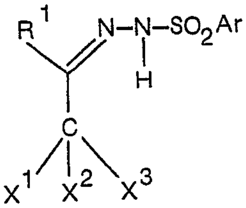

(II)

providing a compound represented by the general formula (I):

M—Y—M′ (I)

[wherein Y represents

—N— or —S—
|
R

(wherein R represents an alkyl group, a 2-alkenyl group, an aralkyl group or an aromatic group); M and M′ represent a hydrogen atom or an alkali metal atom, provided that when Y represents

—N—,
|
R

M and M′ represent a hydrogen atom, and when Y represents —S—, M represents an alkali metal atom; $R^1$ represents a hydrogen atom, an alkyl group or an aromatic group, provided that when Y represents —S—, $R^1$ represents a hydrogen atom; Ar represents an aromatic group; $X^1$ and $X^2$ may each represent a halogen atom, and $X^3$ represents a halogen atom, a hydrogen atom, an alkyl group or an aromatic group, provided that when Y represents, —S—, $X^3$ represents a halogen atom; and when $X^3$ represents a halogen atom, $R^2$ represents a group represented by M—Y— and when $X^3$ represents a hydrogen atom, an alkyl group or an

aromatic group, $R^3$ represents a hydrogen atom, an alkyl group or an aromatic group, respectively] with the proviso that when Y represents

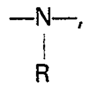

the reaction is carried out in the presence of a base;

combining the compound represented by the general formula (I) and the hydrazone derivative represented by the general formula (II) and allowing a reaction to occur; and

obtaining the 1,2,3-trihetero 5-membered heterocyclic compound represented by the general formula (III).

When the production described above is applied to the production of a 5-mercapto-1,2,3-thiadiazole salt, the present invention comprises the production of a 5-mercapto-1,2,3-thiadiazole salt represented by the general formula (VI):

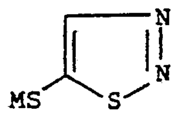

**(VI)**

by allowing a reaction to occur between a hydrazone derivative represented by the general formula (V):

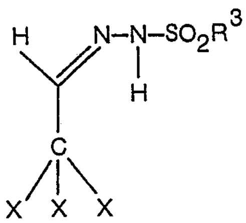

(V)

and a sulfide represented by the general formula (IV):

M—S—M′ (IV)

(wherein $R^3$ is an aryl group; X is a chlorine or bromine atom; M is an alkali metal atom; and M′ is a hydrogen atom or an alkali metal atom).

When the production described above is applied to the production of a 1,2,3-triazole derivative, the present invention comprises the production of a 1,2,3-triazole derivative represented by the general formula (IX):

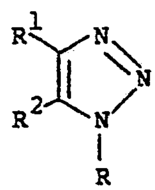

**(IX)**

by allowing a reaction to occur between a hydrazone derivative represented by the general formula (VIII):

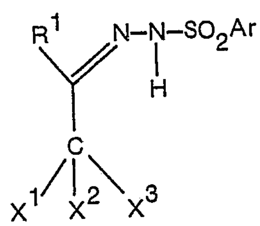

(VIII)

and an amine represented by the general formula (VII):

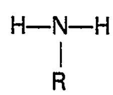

(VII)

in the presence of a base (wherein R is an alkyl group, a 2-alkenyl group, an aralkyl group, or an aromatic

group; $R^1$ is a hydrogen atom, an alkyl group or an aromatic group; Ar is an aromatic group; $X^1$ and $X^2$ may each be a halogen atom and $X^3$ is a halogen atom, a hydrogen atom, and an alkyl group or an aromatic group; and when $X^3$ is a halogen atom, $R^2$ is a group represented by R—NH— and when $X^3$ is a hydrogen atom, an alkyl group or an aromatic group, $R^2$ is a hydrogen atom, an alkyl group or an aromatic group, respectively).

DETAILED DESCRIPTION OF THE INVENTION

The term "1,2,3-trihetero 5-membered heterocyclic compound" is a 5-membered ring compound represented by the general formula (III) wherein three atoms for completing the ring are substituted with atoms selected from the group consisting of a nitrogen atom and a sulfur atom other than a carbon atom.

The starting material of the present invention, i.e., a hydrazone derivative represented by the above general formula (V) is easily obtained by the method described in the literature (K. Bott, *Chem. Ber.,* 1975, *108,* 402). For example, the hydrazone derivatives derived from an acetaldehyde selected from tribromoacetaldehyde or trichloroacetaldehyde and a hydrazone selected from toluenesulfonylhydrazone, benzenesulfonylhydrazone, halobenzenesulfonylhydrazone, nitrobenzenesulfonylhydrazone, naphthylsulfonylhydrazone can be used. The hydrazone derivatives derived from trichloroacetaldehyde and the hydrazone selected from toluenesulfonylhydrazone or benzenesulfonylhydrazone are preferred.

As the sulfide represented by the general formula (IV), sodium sulfide, potassium sulfide, sodium hydrosulfide, potassium hydrosulfide can be used. Sodium sulfide is preferred. The sulfide is used in an amount of not less than 3 molar equivalents to the hydrazone derivatives represented by the above general formula (V), preferably an amount of 3.5 to 5 molar equivalents from economical viewpoint.

The reaction for obtaining the 5-mercapto-1,2,3-thiadiazole salt represented by the general formula (VI) of the present invention is preferably carried out in solvent. The following solvents can be used independently or as mixture: water, alcoholic solvents such as methanol, ethanol, isopropanol, ethylene glycol, and amine solvents such as pyridine, triethylamine.

The reaction for obtaining the 5-mercapto-1,2,3-thiadiazole salt represented by the general formula (VI) of the present invention proceeds smoothly at atmospheric pressure at −10°C to 60°C and is preferably carried out at 0°C to room temperature (about 25°C) owing to the easy operation.

A 5-mercapto-1,2,3-thiadiazole salt represented by the general formula (VI) of the present invention can easily be separated from a salt of sulfinic acid formed as by-product depending on the difference of solubility to alcoholic solvents. Furthermore, the above 5-mercapto-1,2,3-thiadiazole salt can be treated with an alkylating agent such as benzyl chloride or an acylating agent such as acetyl chloride to protect the mercapto group and thus can be isolated as a precursor of the desired 5-mercapto-1,2,3-thiadiazole.

The literature (K. Bott, *Chem. Ber.,* 1975, *108,* 402) discloses that a starting material of this invention, i.e., a hydrazone derivative represented by the general formula (V) can be converted, with the treatment of a base, to a compound represented by the general formula:

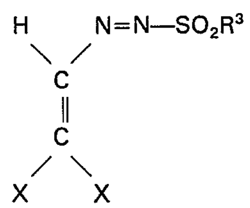

(wherein $R^3$ is an aryl group, X is a chlorine or bromine atom). This compound is also allowed to react with a sulfide represented by the general formula (IV) and a similar reaction to one of this invention proceeds to afford a 5-mercapto-1,2,3-thiadiazole salt represented by the general formula (VI).

A starting material of this invention, i.e., an amine represented by the above general formula (VII) is an industrially easily obtainable compound. For example, the following can be used: alkylamines such as methylamine, ethylamine, propylamine, butylamine, 2-alkenylamines such as 2-propenylamine, 2-butenylamine, 2-pentenylamine, aralkylamines such as benzylamine, α-aminoxylene, p-methoxybenzylamine, and aromatic amines such as aniline, toluidine, chloroaniline, aminoanisole, aminopyridine.

A hydrazone derivative of this invention represented by the above general formula (VIII) can be easily obtained by reacting an α-haloketone with a sulfonylhydrazine. Examples of this compound include α,α-dichloroacetone-tosylhydrazone, 2,2-dichloro-3-butanone-tosylhydrazone, 1,1-dichloro-2-butanonetosylhydrazone, α,α-dichloroacetophenone-tosylhydrazone, chloral-tosylhydrazone, α,α,α-trichloroacetone-tosylhydrazone, α,α-dichloroacetone-benzenesulfonylhydrazone, α,α-dichloroacetone-nitrophenylsulfonylhydrazone, α,α-dichloroacetone-chlorophenylsulfonylhydrazone.

This invention should be carried out in the presence of base when the 1,2,3-triazole derivatives represented by the general formula (IX) are prepared. Excess of an amine represented by the above general formula (VII) can be preferably employed as the base and otherwise, tertiary amines such as triethylamine, pyridine can also be employed as the base.

Use of the solvent is not necessarily required when the 1,2,3-triazole derivatives represented by the general formula (IX) are prepared but, if desired, the following can be used as solvent: water, alcohols such

as methanol, ethanol, ethers such as dioxane, tetrahydrofuran, and mixtures of these solvents.

The reaction for obtaining the 1,2,3-triazole derivatives represented by the general formula (IX) of this invention proceeds at atmospheric pressure at −10°C to 100°C and it is preferably carried out at 0°C to room temperature (about 25°C) for efficient production of the desired product.

The present invention will be further explained in detail by Examples embodying the invention and Reference Examples below.

Example 1

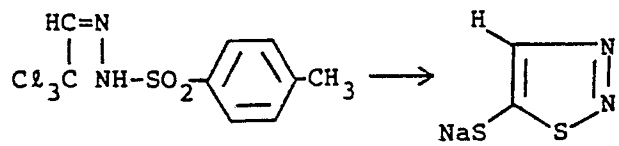

To 50% aqueous ethanol (40 ml) was dissolved 18.0 g (74.9 mmol, 3.75 mol eq) of sodium sulfide ($Na_2S \cdot 9H_2O$) and the solution was stirred under ice cooling. Powdered trichloroacetaldehyde-p-toluenesulfonylhydrazone (6.31 g, 20.0 mmol) was added portionwise to the solution over the period of 5 min. After having been stirred for ca. 10 min, the reaction mixture was allowed to warm to room temperature and further stirred for 1 hr. The solvent was removed at 50°C under reduced pressure and crystals were precipitated when the reaction mixture was concentrated to ca. one-third of its original volume. The mixture was allowed to stand under ice-cooling and the crystals precipitated were collected by filtration. After being dried in a desiccator, the crystals were dissolved in ethanol (100 ml) and treated with active charcoal. The active charcoal was filtered off, the filtrate was concentrated to ca. 50 ml and allowed to stand. Sodium salt of p-toluenesulfinic acid was crystallized and filtered off. The filtrate was further concentrated and allowed to stand to precipitate the (above) sodium salt as crystals which was then filtered off. After confirmation of the absence of p-toluene-sulfinic acid salt by measuring the NMR (spectrum) of one portion of the filtrate, the filtrate was again treated with active charcoal. Removal of the solvent afforded sodium salt of 5-mercapto-1,2,3-thiadiazole (1.68 g) as pale yellow crystals (yield 60%). This product was identical to an authentic sample when their IR spectra were compared. Sodium salt of p-toluenesulfinic acid (3.00 g) was isolated in total (yield 84%).

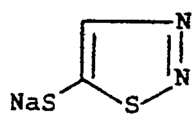

IR spectrum (KBr disc, $cm^{-1}$): 3350, 1665, 1640, 1400, 1200, 1120, 1040, 870, 820.
NMR spectrum ($D_2O$, TSP Deuterated, δ ppm) 8.2 (s).

Example 2

Trichloroacetaldehyde-p-toluenesulfonylhydrazone (3.1 g, 10 mmol) was added portionwise to a 50% aqueous ethanolic solution (50 ml) of 8.4 g of sodium sulfide ($Na_2S \cdot 9H_2O$). After having been stirred at room temperature for 2 hr., the reaction mixture was concentrated under reduced pressure and crystals precipitated were collected by filtration. The crystals were dried over potassium hydroxide under reduced pressure. Isopropanol (17 ml) was added to the crystals. The mixture was stirred at room temperature and undissolved substance was filtered off. The filtrate was treated with active charcoal, heated at 50°C and then the filtrate was filtered. Crystals were precipitated by allowing the filtrate to stand for 16 hr. After removal of the crystals by filtration and evaporation of the solvent under reduced pressure, sodium salt of 5-mercapto-1,2,3-thiadiazole (0.59 g) was obtained as powdery crystals (yield 42%).

Example 3

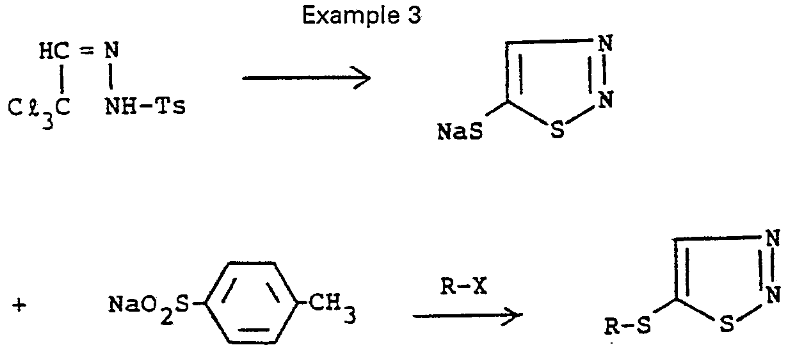


Powdered trichloroacetaldehyde-p-toluenesulfonylhydrazone (6.31 g, 20.0 mmol) was added portionwise to a 50% aqueous ethanolic solution (40 ml) of 15.0 g (62.5 mmol) of sodium sulfide·

($Na_2S \cdot 9H_2O$). After having been stirred at room temperature for 14 hr, the reaction mixture was concentrated to ca. one-third of its original volume under reduced pressure. Crystals precipitated after ice cooling were collected by filtration and dried to give 6.18 g of brown powdery crystals. It is confirmed based on NMR analysis of the aqueous solution of this product that this one is composed of sodium salt of 5-mercapto-1,2,3-thiadiazole and sodium salt of p-toluenesulfinic acid. The following alkylation was carried out using this mixture.

(a) R—X is benzyl bromide

Above mixture (1.00 g) was dissolved into 50% aqueous ethanol (6 ml) and benzyl bromide (806 mg, 4.71 mmol) was added to the solution at room temperature. After 1 hr of stirring, the reaction mixture was extracted with methylene chloride. The organic layer was washed with sat. aq. sodium chloride and dried over sodium sulfate. Evaporation of the solvent afforded 1.123 g of residue. This residue was purifed by column chromatography ($SiO_2$, 30 g, $CH_2Cl_2$) to afford 449 mg of 5-benzylthio-1,2,3-thiadiazole. At the same time, 315 g of benzyl p-tolyl sulfone was isolated.

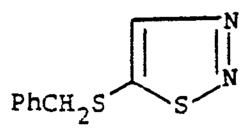

mp 49°C—51°C.

NMR ($CDCl_3$, TMS, δ ppm): 4.14 (2H, s), 7.30 (5H, s), 8.34 (1H, s).

MS (m/e): 208 ($M^+$, 2.3%), 180 (1.5%), 179 (5.3%), 147 (7.8%), 122 (15.1%), 91 (100%).

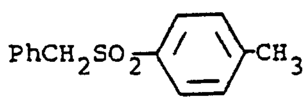

NMR ($CDCl_3$, TMS, δ ppm): 2.39 (3H, s), 4.25 (2H, s), 6.95—7.55 (9H, m).

(b) R—X is benzyl chloride

Above mixture (1.00 g) was dissolved into 50% aqueous ethanol (5 ml) and benzyl chloride (400 mg, 3.2 mmol) was added to the solution under ice cooling. After 2 hr stirring under ice cooling, the reaction mixture was extracted with ether. The ethereal layer was washed with sat. aq. sodium chloride and dried over sodium sulfate. Removal of the solvent under reduced pressure and drying under vacuum accorded 476 mg of 5-benzylthio-1,2,3-thiadiazole alone as crystals.

(c) R—X is ethyl β-bromopropionate

Above mixture (1.00 g) was dissolved into 50% aqueous ethanol (6 ml). Ethyl β-bromopropionate (570 mg) was added to the solution and the whole was stirred for 1 hr. The reaction mixture was extracted with ether. After the ethereal layer was treated in a similar manner as described in (b), excess of ethyl β-bromopropionate was removed under reduced pressure and 343 mg of 5-(2-ethoxycarbonylethyl)thio-1,2,3-thiadiazole alone was obtained as an oil.

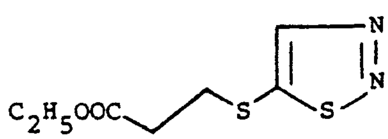

NMR ($CDCl_3$, TMS, δ ppm): 1.29 (3H, t), 2.71 (2H, t), 3.31 (2H, t), 4.17 (2H, q), 8.46 (1H, s).

(d) R—X is bromoethane

Above mixture (500 mg) was dissolved into 50% aqueous ethanol (3 ml). Bromoethane (0.5 ml) was added and the whole was stirred for 1 hr. The reaction mixture was treated in a similar manner as described in (b) to give 133 mg of 5-ethylthio-1,2,3-thiadiazole alone.

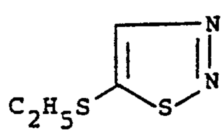

NMR ($CDCl_3$, TMS, δ ppm): 1.43 (3H, t), 3.08 (2H, q), 8.42 (1H, s).

Example 4

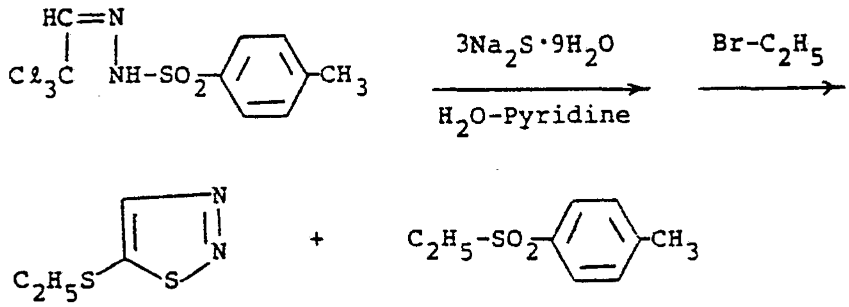


To a 30% aqueous pyridine solution (10 ml) of 721 mg (3.00 mmol) of sodium sulfide ($Na_2S \cdot 9H_2O$) was added powdered trichloroacetaldehyde-p-toluenesulfonylhydrazone (316 mg, 1.00 mmol) and the whole was stirred for 10 hr. To this mixture was added bromoethane (1.0 ml) and the whole was stirred for 4 hr. Then, the reaction mixture was extracted with ethyl acetate. The organic layer was washed with sat. aq. sodium chloride and dried over sodium sulfate. Removal of the solvent under reduced pressure afforded 228 mg of oily substance which was confirmed, based on NMR analysis, to be a mixture of 5-ethylthio-1,2,3-thiadiazole and ethyl p-tolyl sulfide.

Example 5

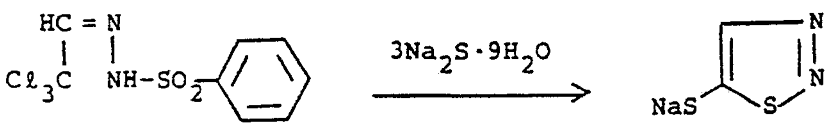


To a 50% aqueous ethanolic solution (100 ml) of 16.8 g (70 mmol) of sodium sulfide ($Na_2S \cdot 9H_2O$) was added portionwise trichloroacetaldehyde-benzenesulfonylhydrazone (6.03 g, 20 mmol) under ice cooling. After having been stirred for 2 hr at room temperature, the reaction mixture was analyzed by NMR spectroscopy to confirm that the mixture contained 30% of 5-mercapto-1,2,3-thiadiazole anion.

Reference Example

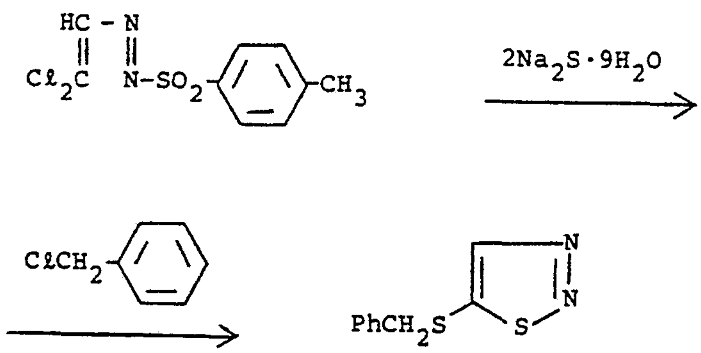


An ethanol (0.5 ml) solution of 1,1-dichloro-2-(p-toluenesulfonylazo)ethene (280 mg, 1.00 mmol) was slowly added to a water (1.0 ml)-ethanol (0.5 ml) solution of 600 mg (2.50 mmol) of sodium sulfide ($Na_2S \cdot 9H_2O$) under ice cooling. After 1 hr stirring at room temperature, the rection mixture was cooled with ice. Benzyl chloride (0.5 ml) was added to the mixture and the whole was stirred for 1 hr. The reaction mixture was extracted with ether. The ethereal layer was washed with sat. aq. sodium hydrogen carbonate and sat. aq. sodium chloride, and then dried over sodium sulfate. After removal of the solvent by distillation, the residual oil was heated at 60°C under vacuum to remove benzyl chloride and 94 mg of 5-benzylthio-1,2,3-thiadiazole was obtained as orange crystals (yield 45%).

Example 6

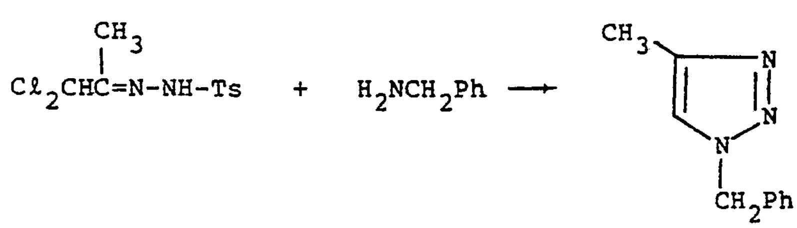

A methanol (5 ml) solution of benzylamine (5.3 g, 50 mmol) was added dropwise to a methanol (10 ml) solution of dichloroacetone-tosylhydrazone (3.0 g, 10 mmol) under ice cooling. After having been stirred for 2.5 hr under ice cooling and for 2 hr at room temperature, the reaction mixture was concentrated under reduced pressure. Benzene (10 ml) was added to the product, the whole was cooled with ice and crystals of benzylamine hydrochloride precipitated was filtered off. The benzene solution was washed with 1N aq. sodium hydroxide and sat. aq. sodium chloride, and then dried over anhydrous magnesium sulfate. Concentration of the solution gave reddish brown oil. The product was purified by silica-gel column chromatography to give 1-benzyl-4-methyl-1H-1,2,3-triazole as pale yellow crystals (1.46 g). Yield 84%.

mp: 61—62°C.

NMR ($CDCl_3$, TMS, δ ppm): 7.37—7.21 (m, 5H), 7.17 (s, 1H), 5.43 (s, 2H), 2.30 (s, 3H).

IR (KBr disk): 3070, 2950, 1500, 1440 $cm^{-1}$.

MS (m/e): 173 ($M^+$).

Example 7

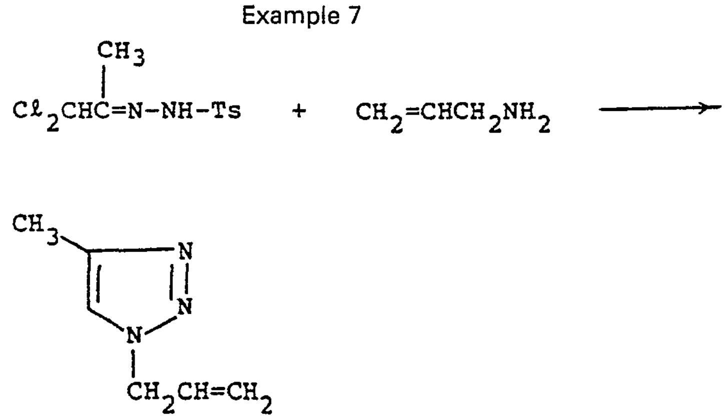

Solid dichloroacetone-tosylhydrazone (3.0 g, 10 mmol) was added to a methanol (15 ml) solution of allylamine (2.85 g, 50 mmol) under ice cooling. The mixture was stirred for 4.5 hr under ice cooling and for 1.5 hr at room temperature. The reaction mixture was concentrated under reduced pressure at room temperature and the product was dissolved with methylene chloride. The solution was washed with 1 N aq. sodium hydroxide and sat. aq. sodium chloride. After having been dried over anhydrous magnesium sulfate, the solution was concentrated under reduced pressure at room temperature to give crude product (1.37 g) as a brown liquid. The crude product was distilled in a micro scale (15 mm Hg, bath temp. 100—160°C) to give 1-allyl-4-methyl-1H-1,2,3-triazole (1.03 g) as a yellow liquid. Yield 83%.

NMR ($CDCl_3$, TMS, δ ppm): 7.30 (d, 1H), 6.00 (m, 1H), 5.25 (m, 2H), 4.90 (d, d, 2H), 2.33 (s, 3H).

IR (neat): 3100, 2950, 1650, 1440, 1210, 1050 $cm^{-1}$.

MS (m/e): 123 ($M^+$).

Example 8

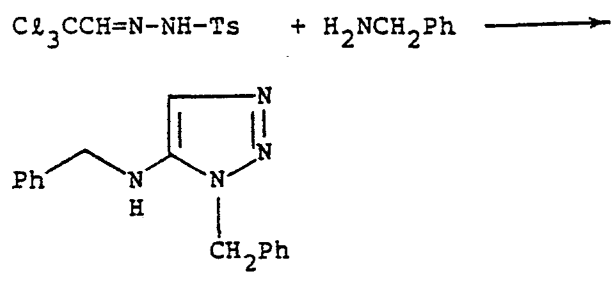

A methanol (5 ml) solution of benzylamine (6.4 g, 60 mmol) was added dropwise to a methanol (10 ml) solution of chloral-tosylhydrazone (3.2 g, 10 mmol) under ice cooling. After having been stirred for 2.5 hr under ice cooling and for 2.5 hr at room temperature, the reaction mixture was concentrated under reduced pressure below 30°C. Benzene was added to the product and crystals of benzylamine hydrochloride precipitated (2.7 g) were filtered off. The benzene solution was washed with 1 N aq. sodium hydroxide and sat. aq. sodium chloride, and then dried over anhydrous magnesium sulfate. After concentration of the solution, the product was purified by silica-gel column chromatography to give 5-benzylamino-1-benzyl-1H-1,2,3-triazole as reddish brown crystals (0.79 g). Yield 30%.

mp: 102—109°C.

NMR (DMSO-$d_6$, TMS, δ ppm): 7.43—7.10 (m, 10H), 6.81 (s, 1H), 6.50 (bt, 1H), 5.41 (s, 2H), 4.25 (bt, 2H).

IR (KBr disk): 3050, 1670, 1600, 1500, 1450, 1290, 1240 $cm^{-1}$.

MS (m/e): 264 ($M^+$).

Example 9

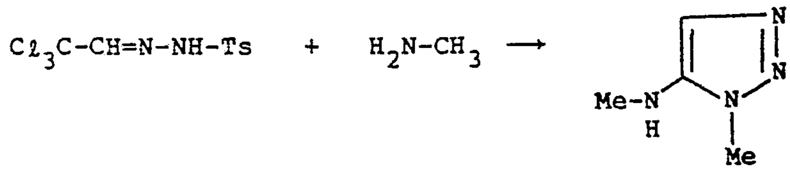

Solid chloral-tosylhydrazone (3.2 g, 10 mmol) was added to a mixture of 40% aqueous methylamine (4.7 g, 60 mmol), water (15 ml) and methanol (10 ml). The mixture was stirred at room temperature for 19 hr. The reaction liquid was saturated with sodium chloride and extracted with chloroform. The extract was washed with sat. aq. sodium chloride, dried over anhydrous magnesium sulfate and concentrated under reduced pressure at room temperature. Brown oil (1.02 g) obtained was purified by silica-gel column chromatography to give 5-methylamino-1-methyl-1H-1,2,3-triazole as pale brown crystals (0.27 g). Yield 25%.

mp: 61—63°C.

NMR ($CDCl_3$, TMS, δ ppm): 6.87 (s, 1H), 4.91 (b.s, 1H), 3.77 (s, 3H), 2.83 (s, 3H).

IR (KBr disk): 3050, 2810, 1440, 1310, 1280 $cm^{-1}$.

MS (m/e): 112 ($M^+$).

While the invention has been described in detail and with reference to specific embodiments thereof, it will be apparent to one skilled in the art that various changes and modifications can be made therein without departing from the spirit and scope thereof.

## Claims

1. A method for preparing a 1,2,3-trihetero 5-membered heterocyclic compound represented by the general formula (III):

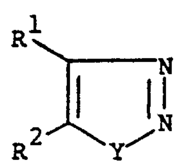

(III)

comprising the steps of:

providing a hydrazone derivative represented by the general formula (II):

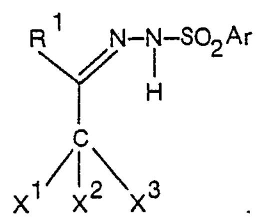

(II)

providing a compound represented by the general formula (I):

M—Y—M′ (I)

[wherein Y represents

—N— or —S—
|
R

(wherein R represents an alkyl group, a 2-alkenyl group, an aralkyl group or an aromatic group); M and M′ represent a hydrogen atom or an alkali metal atom, provided that when Y represents

—N—,
|
R

M and M′ represent a hydrogen atom, and when Y represents —S—, M represents an alkali metal atom; $R^1$

represents a hydrogen atom, an alkyl group or an aromatic group, provided that when Y represents —S—, $R^1$ represents a hydrogen atom, Ar represents an aromatic group; $X^1$ and $X^2$ may each represent a halogen atom, and $X^3$ represents a halogen atom, a hydrogen atom, an alkyl group or an aromatic group, provided that when Y represents —S—, $X^3$ represents a halogen atom; and when $X^3$ represents a halogen atom, $R^2$ represents a group represented by M—Y— and when $X^3$ represents a hydrogen atom, an alkyl group or an aromatic group, $R^2$ represents a hydrogen atom, an alkyl group or an aromatic group, respectively] with the proviso that when Y represents

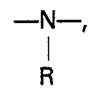

the reaction is carried out in the presence of a base;

combining the compound represented by the general formula (I) and the hydrazone derivative represented by the general formula (II) and allowing a reaction to occur; and

obtaining the 1,2,3-trihetero 5-membered heterocyclic compound represented by the general formula (III).

2. A method for preparing a 1,2,3-trihetero 5-membered heterocyclic compound as claimed in Claim 1, wherein the 1,2,3-trihetero 5-membered heterocyclic compound is a 5-mercapto-1,2,3-thiadiazole salt represented by the general formula (VI):

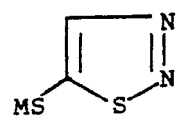

(VI)

and the method comprises allowing a reaction to occur between a hydrazone derivative represented by the general formula (V):

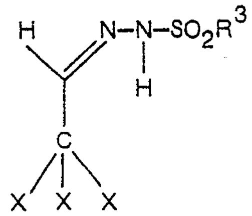

(V)

and a sulfide represented by the general formula (IV):

M—S—M′ (IV)

(wherein $R^3$ is an aryl group; X is a chlorine or bromine atom; M is an alkali metal atom; and M′ is a hydrogen atom or an alkali metal atom).

3. A method for preparing a 1,2,3-trihetero 5-membered heterocyclic compound as claimed in Claim 1, wherein the 1,2,3-trihetero 5-membered heterocyclic compound is a 1,2,3-triazole derivative represented by the general formula (IX):

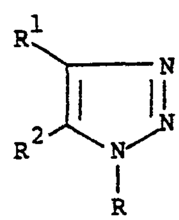

(IX)

and the method comprises allowing a reaction to occur between a hydrazone derivative represented by the general formula (VIII):

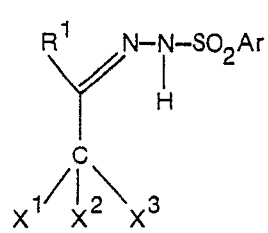

(VIII)

and an amine represented by the general formula (VII):

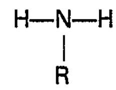 (VII)

in the presence of a base (wherein R is an alkyl group, a 2-alkenyl group, an aralkyl group or an aromatic group; $R^1$ is a hydrogen atom, an alkyl group or an aromatic group; Ar is an aromatic group; $X^1$ and $X^2$ may each be a halogen atom and $X^3$ is a halogen atom, a hydrogen atom, an alkyl group or an aromatic group; and when $X^3$ is a halogen atom, $R^2$ is a group represented by R—NH— and when $X^3$ is a hydrogen atom, an alkyl group or an aromatic group, $R^2$ is a hydrogen atom, an alkyl group or an aromatic group, respectively).

4. A method for preparing a 1,2,3-trihetero 5-membered heterocyclic compound as claimed in Claim 2, wherein the hydrazone derivative represented by the general formula (V) is a compound derived from an acetaldehyde selected from tribromoacetaldehyde or trichloroacetaldehyde and a hydrazone selected from toluenesulfonylhydrazone, benzenesulfonylhydrazone, halobenzenesulfonylhydrazone, nitrobenzenesulfonylhydrazone, or naphthylsulfonylhydrazone.

5. A method for preparing a 1,2,3-trihetero 5-membered heterocyclic compound as claimed in Claim 2, wherein the sulfide represented by the general formula (IV) is a compound selected from the group consisting of sodium sulfide, potassium sulfide, sodium hydrosulfide and potassium hydrosulfide.

6. A method for preparing a 1,2,3-trihetero 5-membered heterocyclic compound as claimed in Claim 5, wherein the sulfide represented by the general formula (IV) is present in an amount of not less than 3 molar equivalents based on the amount of the hydrazone derivative represented by the general formula (V).

7. A method for preparing a 1,2,3-trihetero 5-membered heterocyclic compound as claimed in Claim 6, wherein the sulfide represented by the general formula (IV) is present in an amount in the range of 3.5 to 5 molar equivalents based on the amount of the hydrazone derivative represented by the general formula (V).

8. A method for preparing a 1,2,3-trihetero 5-membered heterocyclic compound as claimed in Claim 2, wherein the reaction is carried out in the presence of a solvent.

9. A method for preparing a 1,2,3-trihetero 5-membered heterocyclic compound as claimed in Claim 8, wherein the solvent is selected from the group consisting of water, alcoholic solvents and amine solvents.

10. A method for preparing a 1,2,3-trihetero 5-membered heterocyclic compound as claimed in Claim 9, wherein the solvent is selected from the group consisting of water, methanol, ethanol, isopropanol, ethylene glycol, pyridine and triethylamine.

11. A method for preparing a 1,2,3-trihetero 5-membered heterocyclic compound as claimed in Claim 2, wherein the reaction is carried out at temperature in the range of −10°C to 60°C.

12. A method for preparing a 1,2,3-trihetero 5-membered heterocyclic compound as claimed in Claim 11, wherein the reaction is carried out at temperature in the range of 0°C to about 25°C.

13. A method for preparing a 1,2,3-trihetero 5-membered heterocyclic compound as claimed in claim 2, wherein the 5-mercapto-1,2,3-thiadiazole salt represented by the general formula (VI) is separated from a salt of sulfinic acid depending on the difference of solubility to alcoholic solvents.

14. A method for preparing a 1,2,3-trihetero 5-membered heterocyclic compound as claimed in Claim 2, wherein the 5-mercapto-1,2,3-thiadiazole salt represented by the general formula (VI) is isolated as a precursor of the 5-mercapto-1,2,3-thiadiazole salt, represented by the general formula (VI), prepared by treating with an alkylating agent or an acylating agent.

15. A method for preparing a 1,2,3-trihetero 5-membered heterocyclic compound as claimed in Claim 2, wherein the hydrazone derivative represented by the general formula (V) is substituted with a compound represented by the general formula:

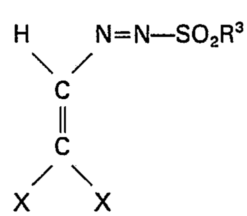

(wherein $R^3$ is an aryl group, X is a chlorine or bromine atom).

16. A method for preparing a 1,2,3-trihetero 5-membered heterocyclic compound as claimed in Claim 3, wherein the amine represented by the general formula (VII) is a compound selected from the group consisting of alkylamines, 2-alkenylamines, aralkylamines and aromatic amines.

17. A method for preparing a 1,2,3-trihetero 5-membered heterocyclic compound as claimed in Claim 16, wherein the amine represented by the general formula (VII) is a compound selected from the group consisting of methylamine, ethylamine, propylamine, butylamine, 2-propenylamine, 2-butenylamine, 2-

pentenylamine, benzylamine, α-aminoxylene, p-methoxybenzylamine, aniline, toluidine, chloroaniline, aminoanisole, and aminopyridine.

18. A method for preparing a 1,2,3-trihetero 5-membered heterocyclic compound as claimed in Claim 3, wherein the hydrazone derivative represented by the general formula (VIII) is a compound selected from the group consisting of α,α-dichloroacetone-tosylhydrazone, 2,2-dichloro-3-butanone-tosylhydrazone, 1,1-dichloro-2-butanone-tosylhydrazone, α,α-dichloroacetophenone-tosylhydrazone, chloral-tosylhydrazone, α,α,α-trichloroacetone-tosylhydrazone, α,α-dichloroacetone-benzenesulfonylhydrazone, α,α-dichloroacetone-nitrophenylsulfonylhydrazone and α,α-dichloroacetone-chlorophenylsulfonylhydrazone.

19. A method for preparing a 1,2,3-trihetero 5-membered heterocyclic compound as claimed in Claim 3, wherein the base is a compound selected from the group consisting of amines represented by the general formula (VII) and tertiary amines.

20. A method for preparing a 1,2,3-trihetero 5-membered heterocyclic compound as claimed in Claim 16, wherein the amine represented by the general formula (VII) is present in excess.

21. A method for preparing a 1,2,3-trihetero 5-membered heterocyclic compound as claimed in Claim 19, wherein the tertiary amine is selected from the group consisting of triethylamine and pyridine.

22. A method for preparing a 1,2,3-trihetero 5-membered heterocyclic compound as claimed in Claim 3, wherein the reaction is carried out in the presence of a solvent.

23. A method for preparing a 1,2,3-trihetero 5-membered heterocyclic compound as claimed in Claim 3, wherein the reaction is carried out at a temperature in the range of −10°C to 100°C.

24. A method for preparing a 1,2,3-trihetero 5-membered heterocyclic compound as claimed in Claim 23, wherein the reaction is carried out at a temperature in the range of 0°C to about 25°C.

**Patentansprüche**

1. Verfahren zur Herstellung einer 5-gliedrigen, heterocyclischen 1,2,3-Trihetero-Verbindung der folgenden allgemeinen Formel (III):

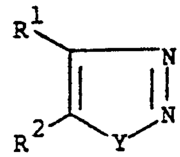

(III)

umfassend folgene Stufen:

Vorsehen eines Hydrazon-Derivats der folgenden allgemeinen Formel (II)

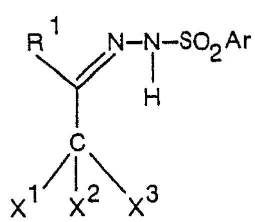

(II)

Vorsehen einer Verbindung der folgenden allgemeinen Formel (I)

M—Y—M′ (I)

worin bedeuten:

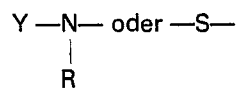

(worin R eine Alkylgruppe, 2-Alkenylgruppe, Aralkylgruppe oder aromatische Gruppe bedeutet); M und M′ ein Wasserstoffatom oder ein Alkalimetallatom, vorausgesetzt daß, wenn Y

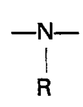

bedeutet, M und M′ ein Wasserstoffatom bedeuten, und wenn Y —S— bedeutet, M ein Alkalimetallatom

bedeutet; $R^1$ ein Wasserstoffatom, eine Alkylgruppe oder eine aromatische Gruppe, vorausgesetzt daß, wenn Y —S— bedeutet, $R^1$ ein Wasserstoffatom bedeutet; Ar eine aromatische Gruppe; $X^1$ und $X^2$ jeweils ein Halogenatom und $X^3$ ein Halogenatom, Wasserstoffatom, eine Alkylgruppe oder eine aromatische Gruppe, vorausgesetzt, daß wenn Y —S— bedeutet, $X^3$ ein Halogenatom bedeutet; und wenn $X^3$ ein Halogenatom bedeutet, steht $R^2$ für eine durch M—Y— angegebene Gruppe, und wenn $X^3$ ein Wasserstoffatom, eine Alkylgruppe oder eine aromatische Gruppe bedeutet, steht $R^2$ für ein Wasserstoffatom, eine Alkylgruppe oder eine aromatische Gruppe, mit der Bedingung, daß wenn Y

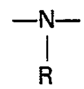

bedeutet, die Umsetzung in Gegenwart einer Base durchgeführt wird;

Zusammenbringen der Verbindung der allgemeinen Formel (I) und des Hydrazon-Derivats der allgemeinen Formel (II) und Eintretenlassen einer Umsetzung und

Gewinnen der 5-gliedrigen, heterocyclischen 1,2,3-Trihetero-Verbindung der allgemeinen Formel (III).

2. Verfahren zur Herstellung einer 5-gliedrigen, heterocyclischen 1,2,3-Trihetero-Verbindung nach Anspruch 1, dadurch gekennzeichnet, daß die 5-gliedrige, heterocyclische 1,2,3-Trihetero-Verbindung ein 5-Mercapto-1,2,3-thiadiazol-Salz der folgenden allgemeinen Formel (VI) ist:

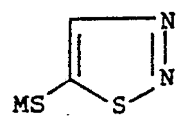

(VI)

und wobei das Verfahren das Eintretenlassen einer Umsetzung zwischen einem Hydrazon-Derivat der folgenden allgemeinen Formel (V):

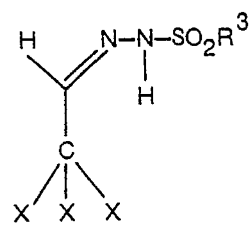

(V)

und einem Sulfid der folgenden allgemeinen Formel (IV):

M—S—M' (IV)

umfaßt, worin $R^3$ eine Arylgruppe, X ein Chlor- oder Bromatom, M ein Alkalimetallatom und M' ein Wasserstoffatom oder ein Alkalimetallatom bedeuten.

3. Verfahren zur Herstellung einer 5-gliedrigen, heterocyclischen 1,2,3-Trihetero-Verbindung nach Anspruch 1, dadurch gekennzeichnet, daß die 5-gliedrige, heterocyclische 1,2,3-Trihetero-Verbindung ein 1,2,3-Triazol-Derivat der folgenden allgemeinen Formel (IX) ist:

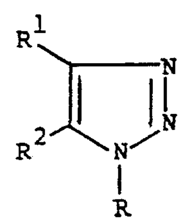

(IX)

und wobei das Verfahren das Eintretenlassen einer Umsetzung zwischen einem Hydrazon-Derivat der folgenden allgemeinen Formel (VIII):

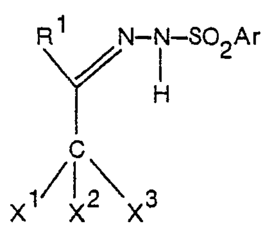

(VIII)

und einem Amin der folgenden allgemeinen Formel (VII):

$$\begin{array}{c} H-N-H \\ | \\ R \end{array} \qquad (VII)$$

in Gegenwart einer Base umfaßt, worin R eine Alkylgruppe, 2-Alkenylgruppe, Aralkylgruppe oder eine aromatische Gruppe, $R^1$ ein Wasserstoffatom, eine Alkylgruppe oder eine aromatische Gruppe, Ar eine aromatische Gruppe, $X^1$ und $X^2$ jeweils ein Halogenatom und $X^3$ ein Halogenatom, Wasserstoffatom, eine Alkylgruppe oder eine aromatische Gruppe bedeuten, und wenn $X^3$ ein Halogenatom bedeutet, $R^2$ eine durch R—NH— dargestellte Gruppe bedeutet, und wenn $X^3$ ein Wasserstoffatom, eine Alkylgruppe oder eine aromatische Gruppe bedeutet, $R^2$ ein Wasserstoffatom, eine Alkylgruppe oder eine aromatische Gruppe bedeutet.

4. Verfahren zur Herstellung einer 5-gliedrigen, heterocyclischen 1,2,3-Trihetero-Verbindung nach Anspruch 2, dadurch gekennzeichnet, daß das durch die allgemeine Formel (V) angegebene Hydrazon-Derivat eine Verbindung ist, die sich ableitet aus einem Acetaldehyd gewählt aus Tribromacetaldehyd oder Trichloracetaldehyd und einem Hydrazon, gewählt aus Toluolsulfonylhydrazon, Benzolsulfonylhydrazon, Halogenbenzolsulfonylhydrazon, Nitrobenzolsulfonylhydrazon oder Naphthylsulfonylhydrazon.

5. Verfahren zur Herstellung einer 5-gliedrigen, heterocyclischen 1,2,3-Trihetero-Verbindung nach Anspruch 2, dadurch gekennzeichnet, daß das durch die allgemeine Formel (IV) angegebene Sulfid eine Verbindung, gewählt aus der Gruppe Natriumsulfid, Kaliumsulfid, Natriumhydrogensulfid und Kaliumhydrogensulfid, ist.

6. Verfahren zur Herstellung einer 5-gliedrigen, heterocyclischen 1,2,3-Trihetero-Verbindung nach Anspruch 5, dadurch gekennzeichnet, daß das durch die allgemeine Formel (IV) angegebene Sulfid in einer Menge von nicht weniger als 3 Mol-Äquivalenten, bezogen auf die Menge des durch die allgemeine Formel (V) angegebenen Hydrazon-Derivats, vorliegt.

7. Verfahren zur Herstellung einer 5-gliedrigen, heterocyclischen 1,2,3-Trihetero-Verbindung nach Anspruch 6, dadurch gekennzeichnet, daß das durch die allgemeine Formel (IV) angegebene Sulfid in einer Menge im Bereich von 3,5 bis 5 Mol-Äquivalenten, bezogen auf die Menge des durch die allgemeine Formel (V) angegebenen Hydrazon-Derivats, vorliegt.

8. Verfahren zur Herstellung einer 5-gliedrigen, heterocyclischen 1,2,3-Trihetero-Verbindung nach Anspruch 2, dadurch gekennzeichnet, daß die Umsetzung in Gegenwart eines Lösungsmittels durchgeführt wird.

9. Verfahren zur Herstellung einer 5-gliedrigen, heterocyclischen 1,2,3-Trihetero-Verbindung nach Anspruch 8, dadurch gekennzeichnet, daß das Lösungsmittel aus der Gruppe Wasser, alkoholische Lösungsmittel und Amin-Lösungsmittel gewählt wird.

10. Verfahren zur Herstellung einer 5-gliedrigen, heterocyclischen 1,2,3-Trihetero-Verbindung nach Anspruch 9, dadurch gekennzeichnet, daß das Lösungsmittel aus der Gruppe Wasser, Methanol, Ethanol, Isopropanol, Ethylenglykol, Pyridin und Triethylamin gewählt wird.

11. Verfahren zur Herstellung einer 5-gliedrigen, heterocyclischen 1,2,3-Trihetero-Verbindung nach Anspruch 2, dadurch gekennzeichnet, daß die Umsetzung bei einer Temperatur im Bereich von −10°C bis 60°C durchgeführt wird.

12. Verfahren zur Herstellung einer 5-gliedrigen, heterocyclischen 1,2,3-Trihetero-Verbindung nach Anspruch 11, dadurch gekennzeichnet, daß die Umsetzung bei einer Temperatur im Bereich von 0 bis etwa 25°C durchgeführt wird.

13. Verfahren zur Herstellung einer 5-gliedrigen, heterocyclischen 1,2,3-Trihetero-Verbindung nach Anspruch 2, dadurch gekennzeichnet, daß das durch die allgemeine Formel (VI) angegebene 5-Mercapto-1,2,3-thiadiazol-salz aus einem Sulfinsäuresalz in Abhängigkeit des Löslichkeitsunterschieds gegenüber alkoholischen Lösungsmitteln abgetrennt wird.

14. Verfahren zur Herstellung einer 5-gliedrigen, heterocyclischen 1,2,3-Trihetero-Verbindung nach Anspruch 2, dadurch gekennzeichnet, daß das durch die allgemeine Formel (VI) angegebene 5-Mercapto-1,2,3-thiadiazol-salz als ein Vorläufer des durch die allgemeine Formel (VI) angegebenen 5-Mercapto-1,2,3-thiadiazol-salzes, hergestellt durch Behandlung mit einem Alkylierungs- oder Acylierungsmittel, isoliert wird.

15. Verfahren zur Herstellung einer 5-gliedrigen, heterocyclischen 1,2,3-Trihetero-Verbindung nach Anspruch 2, dadurch gekennzeichnet, daß das durch die allgemeine Formel (V) angegebene Hydrazon-Derivat mit einer durch die folgende allgemeine Formel angegebenen Verbindung:

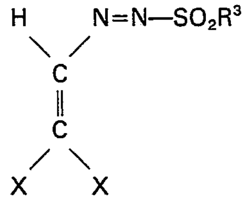

worin $R^3$ eine Arylgruppe und X ein Chlor- oder Bromatom bedeuten, substituiert wird.

16. Verfahren zur Herstellung einer 5-gliedrigen, heterocyclischen 1,2,3-Trihetero-Verbindung nach Anspruch 3, dadurch gekennzeichnet, daß das durch die allgemeine Formel (VII) angegebene Amin eine Verbindung, gewählt aus der Gruppe Alkylamine, 2-Alkenylamine, Aralkylamine und aromatische Amine, ist.

17. Verfahren zur Herstellung einer 5-gliedrigen, heterocyclischen 1,2,3-Trihetero-Verbindung nach Anspruch 16, dadurch gekennzeichnet, daß das durch die allgemeine Formel (VII) angegebene Amin eine Verbindung, gewählt aus der Gruppe Methylamin, Ethylamin, Propylamin, Butylamin, 2-Propenylamin, 2-Butenylamin, 2-Pentenylamin, Benzylamin, α-Aminoxylol, p-Methoxybenzylamin, Anilin, Toluidin, Chloranilin, Aminoanisol und Aminopyridin, ist.

18. Verfahren zur Herstellung einer 5-gliedrigen, heterocyclischen 1,2,3-Trihetero-Verbindung nach Anspruch 3, dadurch gekennzeichnet, daß das durch die allgemeine Formel (VIII) angegebene Hydrazon-Derivat eine Verbindung, gewählt aus der Gruppe α,α-Dichloraceton-tosylhydrazon, 2,2-Dichlor-3-butanon-tosylhydrazon, 1,1-Dichlor-2-butanon-tosylhydrazon, α,α-Dichloracetophenon-tosylhydrazon, Chloral-tosylhydrazon, α,α,α-Trichloraceton-tosylhydrazon, α,α-Dichloraceton-benzolsulfonylhydrazon, α,α-Dichloraceton-nitrophenyl-sulfonylhydrazon und α,α-Dichloraceton-chlorphenyl-sulfonylhydrazon, ist.

19. Verfahren zur Herstellung einer 5-gliedrigen, heterocyclischen 1,2,3-Trihetero-Verbindung nach Anspruch 3, dadurch gekennzeichnet, daß die Base eine Verbindung, gewählt aus der Gruppe Amine der allgemeinen Formel (VII) und tertiäre Amine, ist.

20. Verfahren zur Herstellung einer 5-gliedrigen, heterocyclischen 1,2,3-Trihetero-Verbindung nach Anspruch 16, dadurch gekennzeichnet, daß das durch die allgemeine Formel (VII) angegebene Amin im Überschuß vorliegt.

21. Verfahren zur Herstellung einer 5-gliedrigen, heterocyclischen 1,2,3-Trihetero-Verbindung nach Anspruch 19, dadurch gekennzeichnet, daß das tertiäre Amin aus der Gruppe Triethylamin und Pyridin gewählt wird.

22. Verfahren zur Herstellung einer 5-gliedrigen, heterocyclischen 1,2,3-Trihetero-Verbindung nach Anspruch 3, dadurch gekennzeichnet, daß die Umsetzung in Gegenwart eines Lösungsmittels durchgeführt wird.

23. Verfahren zur Herstellung einer 5-gliedrigen, heterocyclischen 1,2,3-Trihetero-Verbindung nach Anspruch 3, dadurch gekennzeichnet, daß die Umsetzung bei einer Temperatur im Bereich von −10 bis 100°C durchgeführt wird.

24. Verfahren zur Herstellung einer 5-gliedrigen, heterocyclischen 1,2,3-Trihetero-Verbindung nach Anspruch 23, dadurch gekennzeichnet, daß die Umsetzung bei einer Temperatur im Bereich von 0 bis etwa 25°C durchgeführt wird.

## Revendications

1. Un procédé pour préparer un composé hétérocyclique 1,2,3-trihétéroatomique à 5 chaînons représenté par la formule

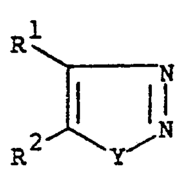

(III)

comprenant les étapes suivantes:

on part d'un dérivé d'hydrazone représenté par la formule générale (II):

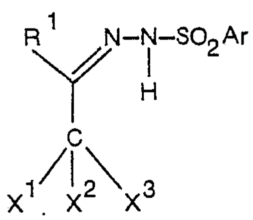

(II)

et un composé représenté par la formule générale (I):

M—Y—M' (I)

[dans lesquelles Y représente

—N— ou —S—,
|
R

(où R représente un groupe alkyle, un groupe 2-alcényle, un groupe arylalkyle ou un groupe aromatique); M et M' représentent un atome d'hydrogène ou de métal alcalin, avec la condition que lorsque Y représente

—N—,
|
R

les symboles M et M' représentent des atomes d'hydrogène et lorsque Y représente —S—, le symbole M représente un atome de métal alcalin; $R^1$ représente un atome d'hydrogène ou un groupe alkyle ou un groupe aromatique, avec la condition que lorsque Y représente —S—, le symbole $R^1$ représente un atome d'hydrogène; Ar représente un groupe aromatique; $X^1$ et $X^2$ peuvent chacun représenter un atome d'halogène et $X^3$ représente un atome d'halogène, un atome d'hydrogène, un groupe alkyle ou un groupe aromatique, avec la condition que lorsque Y représente —S—, le symbole $X^3$ représente un atome d'halogène; et lorsque $X^3$ représente un atome d'halogène, $R^2$ représente un groupe de formule M—Y— et lorsque $X^3$ représente un atome d'hydrogène, un groupe alkyle ou un groupe aromatique, $R^2$ représente un atome d'hydrogène, un groupe alkyle ou un groupe aromatique, respectivement] avec la condition supplémentaire que lorsque Y représente

—N—,
|
R

la réaction est effectuée en présence d'une base;

on combine le composé de formule générale (I) et le dérivé d'hydrazone de formule générale (II) et on laisse se dérouler la réaction; et

on obtient le composé hétérocyclique 1,2,3-trihétéroatomique à 5 chaînons représenté par la formule générale (III).

2. Un procédé pour préparer un composé hétérocyclique 1,2,3-trihétéroatomique à 5 chaînons selon la revendication 1, dans lequel le composé hétérocyclique 1,2,3-trihétéroatomique à 5 chaînons est un sel de 5-mercapto-1,2,3-thiadiazole représenté par la formule générale (VI):

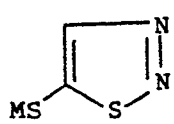

(VI)

et le procédé consiste à mettre à réagir un dérivé d'hydrazone de formule générale (V):

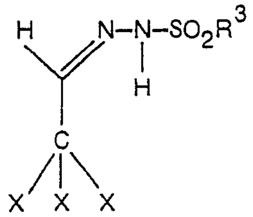

(V)

et un sulfure de formule générale (IV):

M—S—M' (IV)

(dans lesquelles $R^3$ est un groupe aryle; X est un atome de chlore ou de brome; M est un atome de métal alcalin; et M' est un atome d'hydrogène ou de métal alcalin).

3. Un procédé pour préparer un composé hétérocyclique 1,2,3-trihétéroatomique à 5 chaînons selon la revendication 1, dans lequel le composé hétérocyclique 1,2,3-trihétéroatomique à 5 chaînons est un dérivé de 1,2,3-triazole de formule générale (IX):

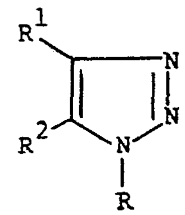

(IX)

et le procédé consiste à mettre à réagir en présence d'une base un dérivé d'hydrazone de formule générale (VIII):

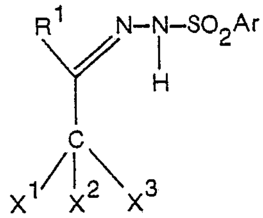

(VIII)

et une amine de formule générale (VII):

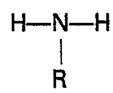

(VII)

(dans lesquelles R est un groupe alkyle, un groupe 2-alcényle, un groupe arylalkyle ou un groupe aromatique; $R^1$ est un atome d'hydrogène, un groupe alkyle ou un groupe aromatique; Ar est un groupe aromatique; $X^1$ et $X^2$ peuvent être chacun un atome d'halogène et $X^3$ est un atome d'halogène, un atome d'hydrogène, un groupe alkyle ou un groupe aromatique; et lorsque $X^3$ est un atome d'halogène, $R^2$ est un groupe de formule R—NH— et lorsque $X^3$ est un atome d'hydrogène, un groupe alkyle ou un groupe aromatique, $R^2$ est un atome d'hydrogène, un groupe alkyle ou un groupe aromatique, respectivement).

4. Un procédé pour préparer un composé hétérocyclique 1,2,3-trihétéroatomique à 5 chaînons selon la revendication 2, dans lequel le dérivé d'hydrazone de formule générale (V) est un composé dérivé d'un acétaldéhyde choisi parmi le tribromoacétaldéhyde et le trichloroacétaldéhyde et une hydrazone choisie parmi la toluènesulfonylhydrazone, la benzènesulfonylhydrazone, les halogénobenzènesulfonylhydrazones, les nitrobenzènesulfonylhydrazones et les naphtylsulfonylhydrazones.

5. Un procédé pour préparer un composé hétérocyclique 1,2,3-trihétéroatomique à 5 chaînons selon la revendication 2, dans lequel le sulfure de formule générale (IV) est choisi parmi le sulfure de sodium, le sulfure de potassium, l'hydrosulfure de sodium et l'hydrosulfure de potassium.

6. Un procédé pour préparer un composé hétérocyclique 1,2,3-trihétéroatomique à 5 chaînons selon la revendication 5, dans lequel le sulfure de formule générale (IV) est présent en quantité de pas moins de 3 équivalents molaires, par rapport à la quantité du dérivé d'hydrazone de formule générale (V).

7. Un procédé pour préparer un composé hétérocyclique 1,2,3-trihétéroatomique à 5 chaînons selon la revendication 6, dans lequel le sulfure de formule générale (IV) est présent en quantité dans l'intervalle de 3,5 à 5 équivalents molaires, par rapport à la quantité du dérivé d'hydrazone de formule générale (V).

8. Un procédé pour préparer un composé hétérocyclique 1,2,3-trihétéroatomique à 5 chaînons selon la revendication 2, dans lequel la réaction est effectuée en présence d'un solvant.

9. Un procédé pour préparer un composé hétérocyclique 1,2,3-trihétéroatomique à 5 chaînons selon la revendication 8, dans lequel le solvant est choisi parmi l'eau, les solvants alcooliques et les solvants aminés.

10. Un procédé pour préparer un composé hétérocyclique 1,2,3-trihétéroatomique à 5 chaînons selon la revendication 9, dans lequel le solvant est choisi parmi l'eau, le méthanol, l'éthanol, l'isopropanol, l'éthylèneglycol, la pyridine et la triéthylamine.

11. Un procédé pour préparer un composé hétérocyclique 1,2,3-trihétéroatomique à 5 chaînons selon la revendication 2, dans lequel la réaction est effectuée à une température dans l'intervalle de −10 à 60°C.

12. Un procédé pour préparer un composé hétérocyclique 1,2,3-trihétéroatomique à 5 chaînons selon la revendication 11, dans lequel la réaction est effectuée à une température dans l'intervalle de 0 à environ 25°C.

13. Un procédé pour préparer un composé hétérocyclique 1,2,3-trihétéroatomique à 5 chaînons selon la revendication 2, dans lequel le sel de 5-mercapto-1,2,3-thiadazole de formule générale (VI) est séparé du

sel d'acide sulfinique en fonction de la différence de solubilité dans les solvants alcooliques.

14. Un procédé pour préparer un composé hétérocyclique 1,2,3-trihétéroatomique à 5 chaînons selon la revendication 2, dans lequel le sel de 5-mercapto-1,2,3-thiadazole de formule générale (VI) est isolé sous forme d'un précurseur du sel de 5-mercapto-1,2,3-thiadiazole de formule (VI), préparé par traitement par un agent alkylant ou un agent acylant.

15. Un procédé pour préparer un composé hétérocyclique 1,2,3-trihétéroatomique à 5 chaînons selon la revendication 2, dans lequel le dérivé d'hydrazones de formule générale (V) est remplacé par un composé de formule générale:

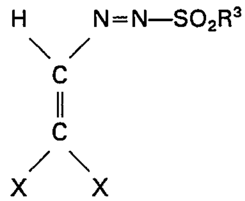

(dans laquelle $R^3$ est un groupe aryle et X est un atome de chlore ou de brome).

16. Un procédé pour préparer un composé hétérocyclique 1,2,3-trihétéroatomique à 5 chaînons selon la revendication 3, dans lequel l'amine de formule générale (VII) est choisie parmi les alkylamines, les 2-alcénylamines, les arylalkylamines et les amines aromatiques.

17. Un procédé pour préparer un composé hétérocyclique 1,2,3-trihétéroatomique à 5 chaînons selon la revendication 16, dans lequel l'amine de formule générale (VII) est choisie parmi la méthylamine, l'éthylamine, la propylamine, la butylamine, la 2-propénylamine, la 2-buténylamine, la 2-penténylamine, la benzylamine, l'α-aminoxylène, la p-méthoxybenzylamine, l'aniline, la toluidine, les chloroanilines, les aminoanisoles et les aminopyridines.

18. Un procédé pour préparer un composé hétérocyclique 1,2,3-trihétéroatomique à 5 chaînons selon la revendication 3, dans lequel le dérivé d'hydrazone de formule générale (VIII) est choisi parmi l'α,α-dichloroacétone-tosylhydrazone, la 2,2-dichloro-3-butanone-tosylhydrazone, la 1,1-dichloro-2-butanone-tosylhydrazone, l'α,α-dichloroacétophénone-tosylhydrazone, la chloral-tosylhydrazone, l'α,α,α-trichloroacétone-tosylhydrazone, l'α,α-dichloroacétone-benzène-sulfonylhydrazone, les α,α-dichloroacétone-nitrophényl-sulfonylhydrazones et les α,α-dichloroacétone-chlorophényl-sulfonylhydrazones.

19. Un procédé pour préparer un composé hétérocyclique 1,2,3-trihétéroatomique à 5 chaînons selon la revendication 3, dans lequel la base est choisie parmi les amines de formule générale (VII) et les amines tertiaires.

20. Un procédé pour préparer un composé hétérocyclique 1,2,3-trihétéroatomique à 5 chaînons selon la revendication 16, dans lequel l'amine de formule (VII) est présente un excès.

21. Un procédé pour préparer un composé hétérocyclique 1,2,3-trihétéroatomique à 5 chaînons selon la revendication 19, dans lequel l'amine tertiaire est choisie parmi la triéthylamine et la pyridine.

22. Un procédé pour préparer un composé hétérocyclique 1,2,3-trihétéroatomique à 5 chaînons selon la revendication 3, dans lequel la réaction est effectuée en présence d'un solvant.

23. Un procédé pour préparer un composé hétérocyclique 1,2,3-trihétéroatomique à 5 chaînons selon la revendication 3, dans lequel la réaction est effectuée à une température dans l'intervalle de −10 à 100°C.

24. Un procédé pour préparer un composé hétérocyclique 1,2,3-trihétéroatomique à 5 chaînons selon la revendication 23, dans lequel la réaction est effectuée à une température dans l'intervalle de 0 à environ 25°C.